# EUROPEAN PATENT APPLICATION

(11) **EP 4 599 805 A1**
(43) Date of publication of application: **13.08.2025**
(21) Application number: 24157220.5
(22) Date of filing: 12.02.2024
(51) Int. Cl.: A61F 5/01

(54) **ANKLE-FOOT-ORTHOSIS**

(71) Applicant: Otto-von-Guericke-Universität Magdeburg, 39106 Magdeburg (DE)
(72) Inventor: Nazha, Hasan, 39106 Magdeburg (DE)
(74) Representative: Schlosser, Martin

(57) **Abstract**

The invention relates to an ankle-foot-orthosis comprising a foot part, a calf part, and a joint mechanically connecting the foot part and the calf part, wherein the joint is detachable from the foot part and the calf part in order to allow both a rigid joint and a flexible joint to be used.

## Description

The invention relates to an ankle-foot-orthosis.

Such an ankle-foot-orthosis can especially be used in order to stabilize a foot relative to a calf of a user. Such stabilization can especially be required in case someone had, for example, an accident, a surgical operation, or another medical condition in the relevant body parts.

It is an object of the invention to provide for an ankle-foot-orthosis that is embodied alternative or better, especially with greater flexibility, compared with known implementations. This is achieved by an ankle-foot-orthosis according to claim 1. Preferred embodiments can, for example, be derived from the dependent claims.

The invention relates to an ankle-foot-orthosis. The ankle-foot-orthosis comprises a foot part, a calf part, and a joint mechanically connecting the foot part and the calf part. The joint is detachable from the foot part and from the calf part so that in a detached state the foot part, the calf part, and the joint are separate from each other.

The described ankle-foot-orthosis comprises three parts that are detachable from each other. This allows significantly increased modularity, especially compared with implementations comprising only one part or only two parts without the possibility to detach the mentioned components without destruction of the parts. Especially, the joint can be replaced by another joint, wherein several different joints can be provided for different purposes. For example, there may be a rigid joint and a flexible joint, especially as described further below. The joints can be replaced and can be used as appropriate for a specific situation. For example, if the foot part should be rigidly fixed relative to the calf part, a rigid joint can be used. This can, for example, be relevant in case a user is sleeping. This rigid joint can be detached from the foot part and the calf part, and can, for example, be replaced by a flexible joint. Such a flexible joint allows a certain movement between the foot part and the calf part, while still connecting them together. The possibility to replace and choose the joints according to a specific situation allows usage of the foot part and the calf part for different situations without having to manufacture the entire ankle-foot-orthosis for each situation separately. The foot part and the calf part can be reused and just the joint is replaced by an appropriate joint for a specific situation.

It should be noted that the foot part, the calf part, and the joint as described herein in any respective implementation can also be regarded as separate inventive aspects, which can also be claimed separately.

A foot part is especially a part covering a region of a human foot. Especially, it can cover the lower and back parts of a foot. The calf part may especially be a part covering a region at a backside of a human calf. The joint is especially any element mechanically connecting the foot part and the calf part. Being detachable especially means that a detaching operation can be performed without destroying any part and without using an extraordinarily increased force. Especially, a detachment operation can be performed without using any separating knife, scissor, or similar tool.

If the parts are separate from each other, this especially means that they do not have any mechanical connection to each other. They can be handled and, if required, replaced separately.

According to an implementation, the joint may be a rigid joint. The rigid joint may rigidly connect the foot part and the calf part. A rigid joint is especially a joint that does not allow relative movement of the foot part relative to the calf part if connected appropriately. Thus, the foot part is rigidly stabilized with respect to the calf part. Such a joint can especially be used in case a user wants to sleep or otherwise to rest, where a relative movement of a foot relative to a calf is not required.

According to an implementation, the joint may be a flexible joint. The flexible joint may flexibly connect the foot part and the calf part. This may especially mean that a certain relative movement or tilting of the foot part relative to the calf part is allowed. The flexible joint can especially be used in case a user wants to walk or otherwise use a foot. Typically, the movement of the foot part relative to the calf part is limited when the flexible joint is used.

According to an implementation, the ankle-foot-orthosis may comprise a further joint that is exchangeable for the joint mechanically connecting the foot part and the calf part. If the joint is a flexible joint, the further joint may be a rigid joint. If the joint is a rigid joint, the further joint may be a flexible joint. This can especially mean that the ankle-foot-orthosis is provided as a set of foot part, calf part, and at least two joints, wherein one joint is a rigid joint and one joint is a flexible joint. Such an ankle-foot-orthosis may also be regarded as an ankle-foot-orthosis arrangement. Typically, only one of the joints is attached at each point in time.

Especially, the flexible joint may comprise a flexible region between respective connection regions to the foot part and to the calf part. The flexible regions may be embodied as a pattern of flexible stripes. Such a flexible region may provide for the necessary flexibility between foot part and calf part. Flexible stripes may be used in order to ensure the required flexibility and further properties, for example as described further below.

Especially, the pattern in the flexible region may be embodied as a plurality of unit cells, especially but not necessarily of identical unit cells. Such unit cells may each have a certain form of its flexible stripes, which may repeat identically in case of identical unit cells. If unit cells are used, which are not identical, each unit cell may have a certain form of its flexible stripes, which can provide for specific properties.

The pattern of flexible stripes may especially span along an entire extension of the joint transverse to a line or a thought connection between the foot part and the calf part. This may especially be the shortest line or thought connection. Thus, the entire extension of the joint may be used in order to provide for flexibility and possibly for further properties, for example as described below.

The flexible joint, or a flexible region of the flexible joint, may especially be made of thermoplastic polyurethane (TPU), thermoplastic copolyester (TPC), and/or a combination of thermoplastic polyester and thermoplastic polyurethane. Such materials have been proven suitable for typical applications. However, also other materials can be used, especially materials that are flexible and which can, for example, have a forceless ground state.

Any joint, the foot part and/or the calf part may be provided with a biocompatible internal padding, which provides for a contact to a human body. In this case, materials used could be any stiff material for the calf part and the foot part, and any stiff or elastic material for the joint. In a case without such a biocompatible internal padding, the materials used for the calf part, the foot part, and/or the joint may especially be any biocompatible stiff material for the calf part and the foot part, and any biocompatible stiff or elastic material for the joint part. A biocompatible material is especially a material that does not lead to harm or irritation at a contact with a human skin.

Especially, the flexible joint, or a flexible region of the flexible joint, may have auxetic properties, a negative Poisson's ratio, negative elasticity, and/or negative compressibility. This may especially mean that if such a material is extended along a certain line, for example a line being transverse to a line connecting the foot part and the calf part, it extends in a direction transverse to the line along which it is extended. This is a property of certain materials and especially a property that may be reached by using certain unit cells, for example unit cells that are described further below with respect to the drawings. Such auxetic properties have been proven suitable for typical applications, because they provide for specifically good support of a foot and a calf of a human when wearing the ankle-foot-orthosis.

The joint or the further joint may especially comprise a first connection region for connecting to the foot part and/or a second connection region for connecting to the calf part. Such connection regions may especially be embodied in order to provide for a secure connection, which is, however, detachable so that the joint may be detached from the foot part and/or the calf part.

Especially, the first connection region may be adapted to engage and disengage connection by rotation of the joint relative to the foot part. The second connection region may be adapted to engage and disengage connection by rotation of the joint relative to the calf part. Thus, connection may be engaged and disengaged just by rotating parts relative to each other.

According to an implementation, the first connection region comprises one or more tongues and the foot part comprises one or more grooves for receiving the tongues. According to an implementation, the second connection region comprises one or more tongues and the calf part comprises one or more grooves for receiving the tongues. According to an implementation, the foot part comprises one or more tongues and the first connection region comprises one or more grooves for receiving the tongues. According to an implementation, the calf part comprises one or more tongues and the second connection region comprises one or more grooves for receiving the tongues. With such a combination of tongues and grooves in one or more of the described implementations, a simple but reliable connection that can be engaged and disengaged by rotation can be achieved.

Especially, at least one connection region, the foot part and/or the calf part may comprise two or more tongues and/or two or more grooves, such that from a disconnected state at least one tongue can be interposed between two grooves or at least one groove can be interposed between two tongues, and can be connected by relative rotation. This allows for engaging and disengaging the connection between the joint and one of the parts by rotation, wherein the rotation does not have to be performed along the entire extension of the joint, but only along a part of that extension. For example, only a relative rotation of about 90° may be sufficient in order to engage and disengage the connection. Especially, a groove may only be regarded as a groove if it is able to surround a tongue such that the tongue cannot be removed perpendicular to a plane of rotation or perpendicular to an extension of the groove.

According to an implementation, the first connection region and the foot part may be held in a static relative state by friction. According to an implementation, the second connection region and the calf part may be held in a static relative state by friction. Such friction may, especially in some implementations, be sufficient in order to provide for sufficient security against disengaging the joint from the foot part and/or the calf part involuntarily.

According to an implementation, the first connection region and the foot part may be held in a static relative state by one or more removable pins and/or pads. Especially, a pin may be a small object and/or a lengthy object providing for a form-fitting connection.

It may be removed in order to disengage the connection. A pad may especially be a pad comprising an adhesive surface, which may connect the connection region or the joint with a part. It may be removed in order to disengage the connection. Correspondingly, the second connection region and the calf part may be held in a static relative state by one or more removable pins and/or pads.

Especially, a static relative state may mean that no rotation between components is possible, and thus no detachment is possible.

Especially, the implementation described herein may provide for a seamless transition between static and dynamic modes. It may facilitate dorsiflexion and plantarflexion movements in dynamic situations. It can significantly reduce production costs through dynamic mode adaptability. Manufacturing processes like molding, extrusion, casting, or injection may be streamlined. This may reduce the costs for end users. Static and dynamic devices may be separately replaced. Prescription and fitting processes may be simplified. Time, effort and costs associated with multiple devices can be saved. Biocompatible materials for prolonged skin contact can be used without irritation. Customizable material properties can be used for versatility. The use of auxetic mechanical metamaterials may reduce waste. Reliance on auxetic materials for smart properties may balance motion and stability. Biocompatible materials may provide for long-term comfort and safety. The implementation may be customizable for individual patient needs. The implementation may accommodate a wide range of patient sizes and specific demands. The implementation may address practical challenges through adaptability. The implementation may be applicable to abnormal gate, rehabilitation, post-injury, and post-surgery situations. A user-friendly design may be implemented. For example, an internal padding, for example made of foam, for additional comfort and skin irritation prevention may be used. Straps may be used for stability and enhanced functionality. A holistic approach may be used for patient comfort and usability.

The unit cells which have already been mentioned above can especially be embodied of, or can comprise, flexible straps. They can work as directional and angular limiting mechanism, especially having the feature that controls and limits the range of movement of rotation of the ankle-foot-orthosis components to specific directions and/or within certain angular limits.

Especially, the ankle-foot-orthosis may include a replaceable joint that allows it to be used in both static and dynamic situations. This replaceable joint may be a key feature, it can be used in a static situation (when the patient lies down to replace a solid or night splint orthosis) or in a dynamic situation (for use during day activities) according to the patient's need as it enables the ankle-foot-orthosis to perform both dorsiflexion and plantarflexion movements in the dynamic case by the help of the specific mentioned auxetic mechanical metamaterial structure design, and provides the stability for the foot in the static case.

The dynamic joint may be made from auxetic mechanical metamaterial. This material is capable of achieving smart properties that allow it to deform in dorsiflexion and plantarflexion directions, enabling the AFO to function dynamically. Smart unit cells of the auxetic mechanical metamaterial have the shape of a parallelogram with curved ends and are connected to each other, ensuring that they perform both dorsiflexion and plantarflexion movements.

The ankle-foot-orthosis may be made of biocompatible materials, that are safe for prolonged contact with human skin, reducing the risk of skin irritation. In addition, it can be manufactured by using different materials, taking into account the design considerations for the stiffness of the calf and foot parts and the elasticity of the joint especially for the dynamic case, and add foam as internal padding to provide more convenience to the patients.

The ankle-foot-orthosis can be designed with desired dimensions and can be easily modified to fit individual patients or specific patient groups. In addition, the novel ankle-foot-orthosis and its joint part that is designed with auxetic metamaterials could be modified in dimensions and manufacturing materials to support a wide range of patient sizes and demands.

The ankle-foot-orthosis with a replaceable joint (two in one) may be designed to reduce costs and manufacturing time, making it a more economical option. For mass production, manufacturing methods as well could be molding, extrusion, casting, or injection techniques.

The ankle-foot-orthosis can be used for various conditions, including supporting patients with abnormal gate, rehabilitation, and post-injury or post-surgery situations. Foam can be used as internal padding to provide more convenience to the patients or the biocompatible materials mentioned in general could be a good choice to avoid irritation in the long term of ankle-foot-orthosis use. Straps can be used to provide more stability when using it. Aimed for ease of use by assembling (mounting and twisting) the joint tongues with grooves of the calf and foot parts, or wise versa, friction may secure stability. In addition, small sticky pads can be used to increase the assembly stability of the parts and prevent them from sliding out of each other.

The implementation of an ankle-foot-orthosis with a replaceable joint can especially be designed and manufactured so that the device can be used in the static situation when the static joint is installed, or it can be used in the dynamic situation when the dynamic joint is installed to perform the dorsiflexion and plantarflexion movements. This dynamic joint may be designed based on the auxetic mechanical metamaterial, which is defined as a material designed to have a property not found in conventional materials. They are designed from groups of multiple elements (unit cells) arranged in repeating patterns that are capable of realizing the smart properties to achieve benefits beyond what is possible with conventional materials. Thus, the components of the proposed ankle-foot-orthosis can be obtained for use in a static situation (when the patient lies down to replace a solid or night splint orthosis) or a dynamic situation (for use during daily activities) according to the patient's needs. This means replacing the purchase of two ankle-foot-orthoses to perform the above-mentioned human functions by purchasing one ankle-foot-orthosis (with a replaceable joint) that performs the same functions and is manufactured in a relatively shorter time and with affordable materials. This means saving time, effort, and money to achieve the desired purpose with higher efficiency (one device with a replaceable joint instead of two devices).

The ankle-foot-orthosis may especially have a replaceable joint so that the device can be used in the static situation when the static joint is installed or used in a dynamic state when using the dynamic joint, or flexible joint, in the direction of dorsiflexion and plantarflexion by taking advantage of the design of auxetic mechanical metamaterial smart unit cells that can move in both directions. The novel device may be made of materials that are biocompatible with human skin, meaning that it does not cause any skin irritation or inflammations when used in the long term. The new device, the joint, and/or the parts can be manufactured by any method or technology, such as molding, 3D printing, injection, extrusion, etc. A static joint may be installed to use the device while lying down to replace the solid or night splint orthosis, or the dynamic joint can be installed for use during daily activities according to the patient's need. The novel design may replace the purchase of two orthotic devices to perform the human functions mentioned above by purchasing one ankle-foot-orthosis with a replaceable joint that performs the same function and is manufactured in a relatively shorter time and with affordable materials. This means saving time, effort, and money to achieve the desired purpose with higher efficiency (one device with a replaceable joint instead of two devices). For example, the joint-replaceable ankle-foot-orthosis can be used to support patients with abnormal gait, as well as for rehabilitation. The novel ankle-foot-orthosis can be designed with the desired dimensions and easily modified to suit the case of one patient with a specific size, or with dimensions and sizes suitable for a specific group of patients (small-medium-large) according to sizes of the feet of this particular target group of patients.

In the prior art, the working principle of orthoses is to perform the functions as static only or dynamic only. In contrast thereto, the ankle-foot-orthosis described herein with a replaceable joint was designed and manufactured so that the device can be used in the static situation when the static joint is installed, or it can be used in the dynamic situation when the dynamic or flexible joint is installed to perform the dorsiflexion and plantarflexion movements. The dynamic joint may be designed based on the auxetic mechanical metamaterial, which is defined as a material designed to have a property not found in conventional materials. They are designed from groups of multiple elements (unit cells) arranged in repeating patterns that are capable of realizing the smart properties to achieve benefits beyond what is possible with conventional materials. Thus, the components of the proposed ankle-foot-orthosis can be obtained for use in a static situation (when the patient lies down to replace a solid or night splint orthosis) or in a dynamic situation (for use during daily activities) according to the patient's need. This means replacing the purchase of two ankle-foot-orthoses to perform the above-mentioned human functions by purchasing one ankle-foot-orthosis (with a replaceable joint) that performs the same function and is manufactured in a relatively shorter time and with affordable materials. This saves time, effort, and money to achieve the desired purpose with higher efficiency (one device with a replaceable joint instead of two devices). A unit cell can, for example, be designed by computer-aided design. It can especially be a two-dimensional sketch or can be a two-dimensional structure. For example, extruding can be used for manufacturing.

Further details and advantages will be apparent from the following description of the drawings, wherein:
- fig. 1:: shows an ankle-foot-orthosis with a flexible joint,
- fig. 2:: shows a flexible joint,
- fig. 3:: shows a calf part,
- fig. 4:: shows a foot part,
- fig. 5:: shows a pad,
- fig. 6:: shows an ankle-foot-orthosis with a rigid joint,
- fig. 7:: shows a rigid joint, and
- fig. 8:: shows different patterns made of unit cells.

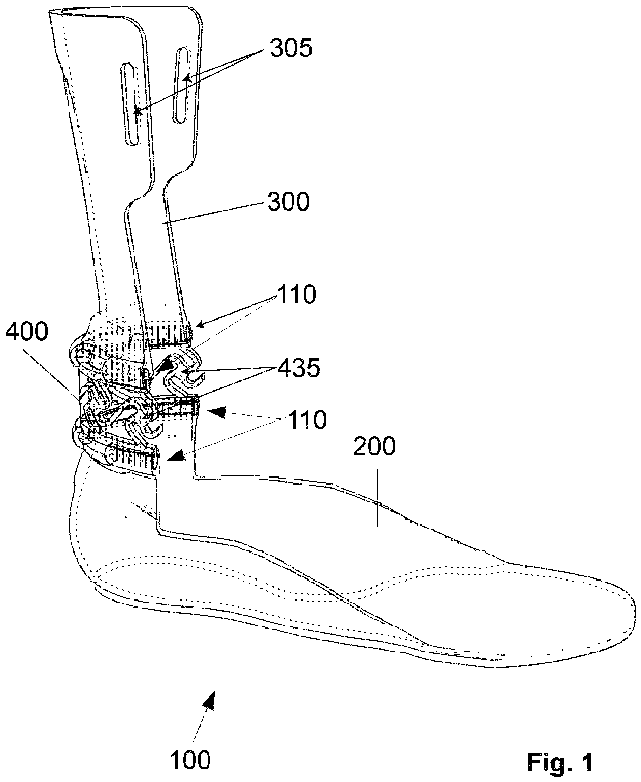

Fig. 1 shows an ankle-foot-orthosis 100 according to an embodiment. The ankle-foot-orthosis comprises a foot part 200, a calf part 300, and a joint 400 mechanically connecting the foot part 200 and the calf part 300.

The foot part 200 is adapted to cover an outer area of a human foot. The calf part 300 is adapted to cover an outer area of a human calf. This is immediately apparent from Fig. 1. The joint 400 mechanically connects the foot part 200 and the calf part 300 in order to secure their relative position, but allows a tilt movement of the foot part 200 relative to the calf part 300.

The calf part 300 comprises two holes 305 that can be used in order to secure straps that can be positioned around a front part of a human calf. This can be used in order to fix the calf part 300 to a human calf.

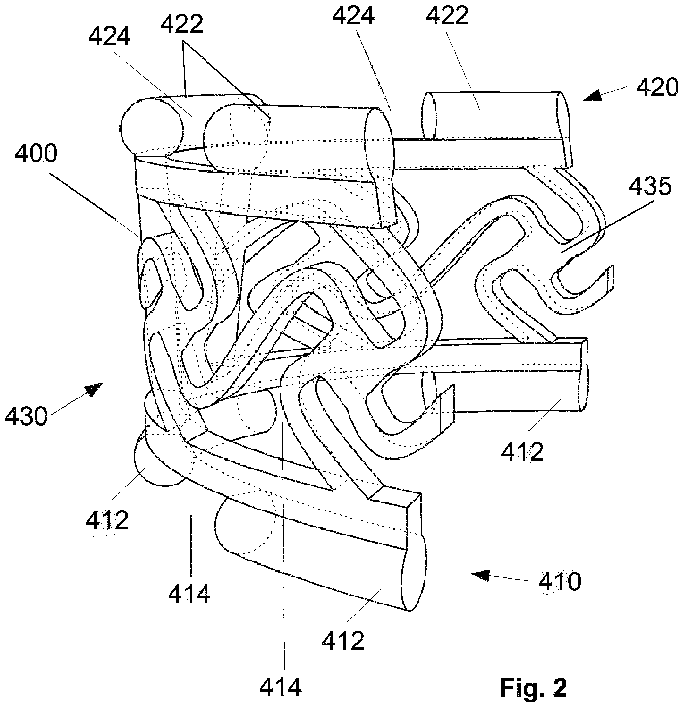

The joint 400 comprises a plurality of unit cells 435. This will be further explained below with respect to fig. 2. These unit cells 435 ensure that the joint 400 has auxetic properties.

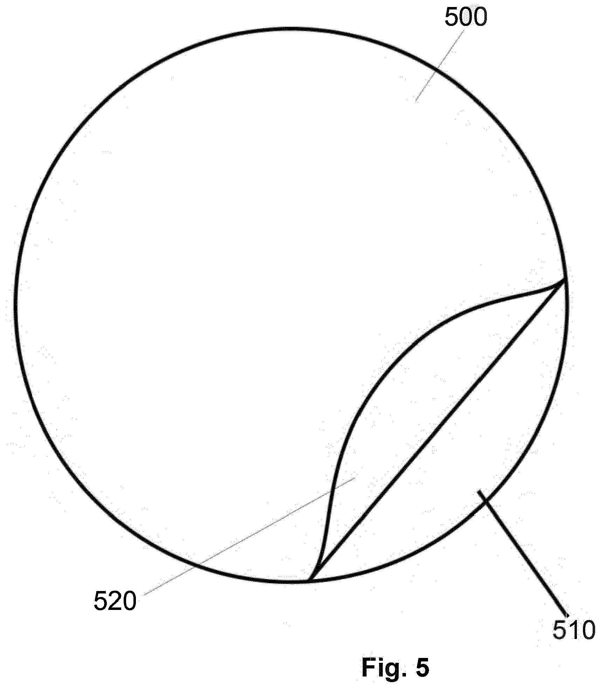

In fig. 1, there are marked four fixing positions 110, where pads can be used in order to secure the relative position of the joint 400, the calf part 300 and the foot part 200. For example, pads with an adhesive surface as shown in fig. 5 can be used. However, such pads are not shown in fig. 1.

The joint 400, the calf part 300 and the foot part 200 can be separated from each other by using means that will be explained further below.

Fig. 2 shows the joint 400 in further detail. The joint 400 has a first connection region 410, a second connection region 420 and a flexible region 430 between the connection regions 410, 420. The flexible region 430 is embodied with a plurality of unit cells 435 made of flexible stripes, which form a pattern. These unit cells 435 ensure that the flexible region 430 has auxetic properties, as already described further above. In the present case, the unit cells 435 are identical.

The first connection region 410 is adapted to connect the joint 400 to the foot part 200. The second connection region 420 is adapted to connect the joint 400 to the calf part 300. The first connection region 410 comprises three tongues 412, which are arranged along an approximately half-circular line with two intermittences 414 between the tongues 412. Correspondingly, the second connection region 420 comprises three tongues 422 arranged along an approximately half-circular line with two intermittences 424 between the tongues 422.

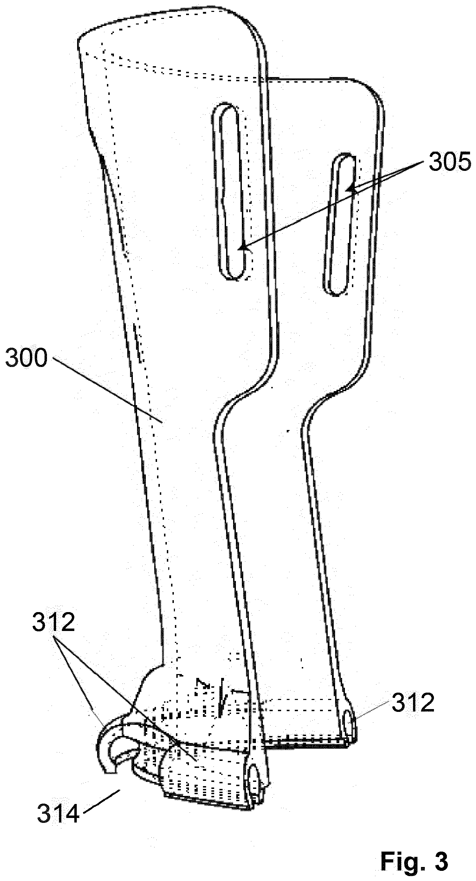

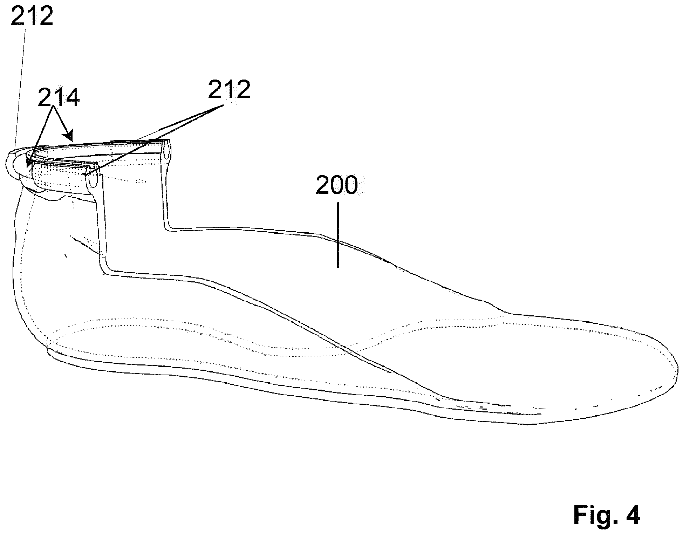

Fig. 3 shows the calf part 300 in greater detail. The calf part 300 comprises, at its lower side, three grooves 312 which are arranged complementarily to the tongues 422 of the second connection region 420 of the joint 400. Between these three grooves 312, there are two intermittences 314, of which one is visible in fig. 3. Correspondingly, fig. 4 shows the foot part 200 in greater detail. At its upper side, it comprises three grooves 212 arranged complementarily to the tongues 412 of the first connection region 410 of the joint 400. The grooves 212 are intermitted by two intermittences 214.

The described implementation of the foot part 200, the calf part 300, and the joint 400 allows engagement and disengagement of a connection between the joint 400 and the foot part 200 and/or the calf part 300 by rotating the elements, wherein by rotating a tongue 412, 422 is inserted in a corresponding groove 212, 312 for engaging a connection, and is brought out of the groove 212, 312 also by rotating. Due to the intermittences 214, 314, 414, 424, it is not necessary to rotate respective parts by 180°, but only by about 60°, because the tongues 412, 422 can be placed in intermittences 214, 314 of the foot part 200 and the calf part 300, and then a relative rotation can engage a connection. The same is true for disengaging the connection.

Fig. 5 shows a pad 500 that can be used in order to secure the connection in addition to any securing of the connection by friction. The pad 500 comprises an adhesive surface 510 which is covered, in its ground state, by a cover 520. The cover 520 can be removed, so that the adhesive surface 510 is exposed. This adhesive surface 510 can be put to the fixing positions 110, which are shown in fig. 1. There, they connect material of the foot part 200 and the joint 400, or of the calf part 300 and the joint 400. Relative rotation of these elements is thus prevented by using the pads 500. Such pads 500 can also easily be removed in order to allow rotation.

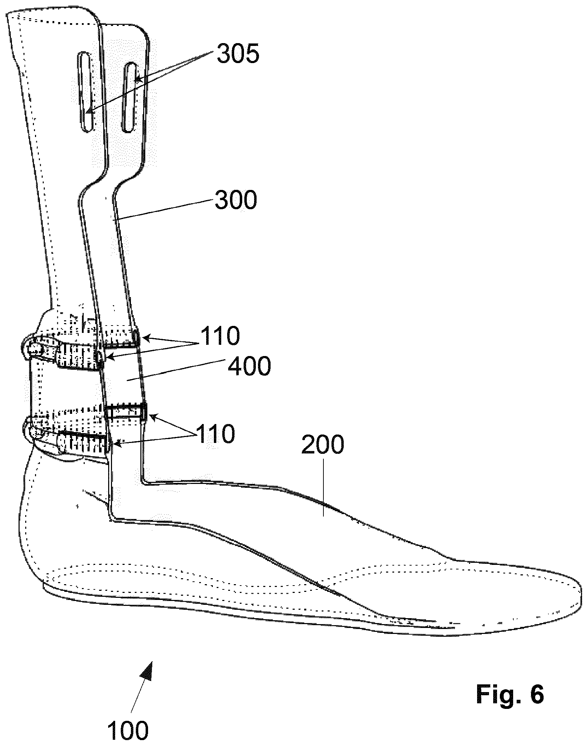

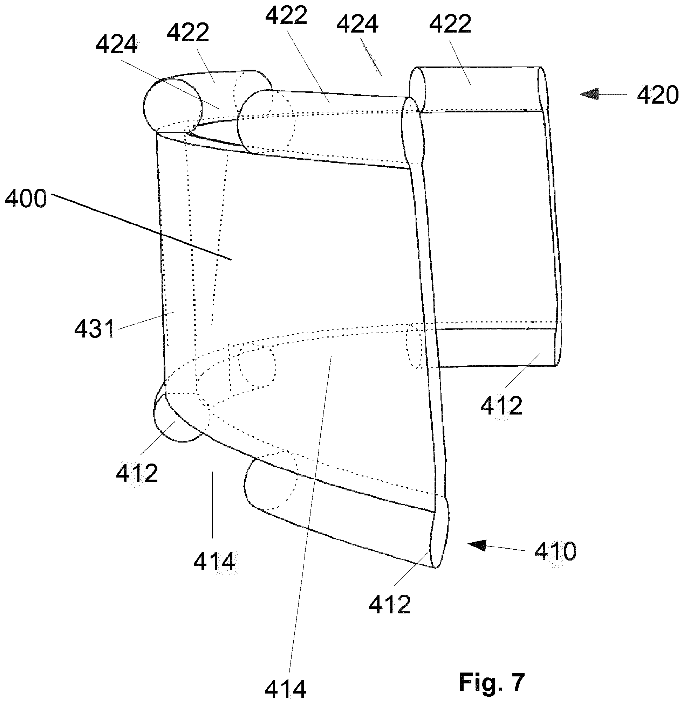

Fig. 6 shows an ankle-foot-orthosis according to a second embodiment, which is only partially different from the first embodiment. In contrast to the first embodiment, the ankle-foot-orthosis 100 of fig. 6 does not have a flexible joint, but a rigid joint 400. This rigid joint is shown in further detail in fig. 7. The connection regions 410, 420 are embodied identically to the flexible joint 400 shown in fig. 2. However, in contrast thereto, the rigid joint 400 of fig. 7 comprises a rigid region 431 between the two connection regions 410, 420. The rigid region 431 is made of a stiff material and ensures that the foot part 200 and the calf part 300 are not tiltable against each other.

It should be noted that the two shown versions of a joint 400, namely the rigid joint and the flexible joint, can be exchanged arbitrarily, so that the foot part 200 and the calf part 300 can be connected either by a rigid joint or a flexible joint. Thus, the joint 400 can be adapted to the currently desired properties of the ankle-foot-orthosis.

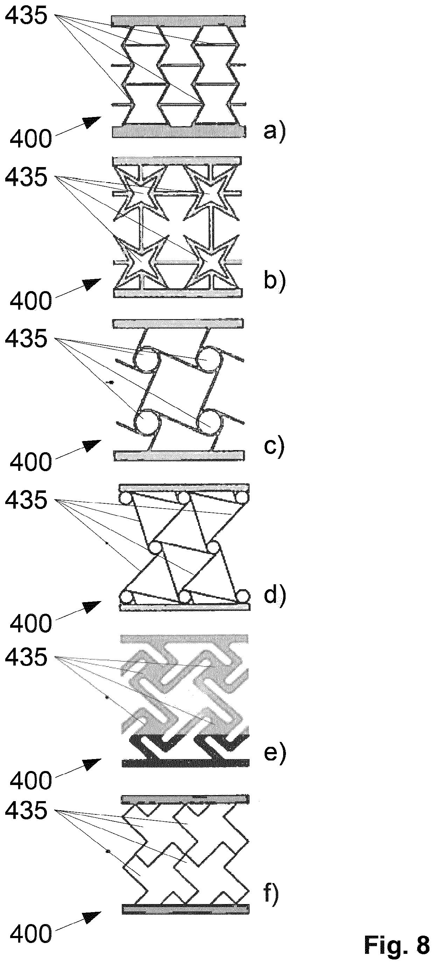

Figs. 8a to 8f show six different patterns of a possible flexible region 430 of a flexible joint 400. In each case, four unit cells 435 are shown, which may be repeated indefinitely. All of these different unit cells 435 have auxetic properties, meaning that in case a force is applied extending the pattern in the horizontal direction of fig. 8, an extension in vertical direction is induced. The pattern shown in fig. 8e is also shown in fig. 2. However, also the other shown patterns may be used and may yield slightly different properties.

It is to be noted that features may be described in combination in the claims and in the description, for example in order to provide for better understandability, despite the fact that these features may be used or implemented independent from each other. The person skilled in the art will note that such features can be combined with other features or feature combinations independent from each other.

References in dependent claims may indicate preferred combinations of the respective features, but do not exclude other feature combinations.

### List of reference signs

- 100: ankle-foot-orthosis
- 110: fixing positions
- 200: foot part
- 212: grooves
- 214: intermittences
- 300: calf part
- 305: holes
- 312: grooves
- 314: intermittences
- 400: joint
- 410: first connection region
- 412: tongues
- 414: intermittences
- 420: second connection region
- 422: tongues
- 424: intermittences
- 430: flexible region
- 431: rigid region
- 435: unit cells
- 500: pad
- 510: adhesive surface
- 520: cover

## Claims

1. Ankle-foot-orthosis (100) comprising
- a foot part (200),
- a calf part (300), and
- a joint (400) mechanically connecting the foot part (200) and the calf part (300), wherein
- the joint (400) is detachable from the foot part (200) and from the calf part (300) so that in a detached state the foot part (200), the calf part (300), and the joint (400) are separate from each other.

2. Ankle-foot-orthosis (100) according to claim 1,
- wherein the joint (400) is a rigid joint (400), rigidly connecting the foot part (200) and the calf part (300).

3. Ankle-foot-orthosis (100) according to claim 1,
- wherein the joint (400) is a flexible joint (400), flexibly connecting the foot part (200) and the calf part (300).

4. Ankle-foot-orthosis (100) according to one of claims 2 or 3,
- further comprising a further joint (400) that is exchangeable for the joint (400) mechanically connecting the foot part (200) and the calf part (300),
- wherein the further joint (400) is a rigid joint (400) if the joint (400) is a flexible joint (400), and wherein the further joint (400) is a flexible joint (400) if the joint (400) is a rigid joint (400).

5. Ankle-foot-orthosis (100) according to one of claims 3 or 4,
- wherein the flexible joint (400) comprises a flexible region (430) between respective connection regions (410, 420) to the foot part (200) and the calf part (300),
- wherein the flexible region (430) is embodied as a pattern of flexible stripes.

6. Ankle-foot-orthosis (100) according to claim 5,
- wherein the pattern in the flexible region (430) is embodied as a plurality of unit cells (435), or of identical unit cells (435).

7. Ankle-foot-orthosis (100) according to one of claims 5 or 6,
- wherein the pattern of flexible stripes spans along an entire extension of the joint (400) transverse to a line between the foot part (200) and the calf part (300).

8. Ankle-foot-orthosis (100) according to one of claims 3 to 7,
- wherein the flexible joint (400), or a flexible region (430) of the flexible joint (400), is made of thermoplastic polyurethane (TPU), thermoplastic copolyester (TPC), and/or a combination of thermoplastic polyester and thermoplastic polyurethane.

9. Ankle-foot-orthosis (100) according to one of claims 3 to 8,
- wherein the flexible joint (400), or a flexible region (430) of the flexible joint (400), has auxetic properties, a negative Poissons's ratio, negative elasticity, and/or negative compressibility.

10. Ankle-foot-orthosis (100) according to one of the preceding claims,
- wherein the joint (400) or the further joint (400) comprises a first connection region (410) for connection to the foot part (200) and/or a second connection region (420) for connecting to the calf part (300).

11. Ankle-foot-orthosis (100) according to claim 10,
- wherein the first connection region (410) is adapted to engage and disengage connection by rotation of the joint (400) relative to the foot part (200), and/or
- wherein the second connection region (420) is adapted to engage and disengage connection by rotation of the joint (400) relative to the calf part (300).

12. Ankle-foot-orthosis (100) according to one of claims 10 or 11,
- wherein the first connection region (410) comprises one or more tongues (412) and the foot part (200) comprises one or more grooves (212) for receiving the tongues (412),
and/or
- wherein the second connection region (420) comprises one or more tongues (422) and the calf part (300) comprises one or more grooves (312) for receiving the tongues,
and/or
- wherein the foot part (200) comprises one or more tongues and the first connection region (410) comprises one or more grooves for receiving the tongues,
and/or
- wherein the calf part (300) comprises one or more tongues and the second connection region (420) comprises one or more grooves for receiving the tongues.

13. Ankle-foot-orthosis (100) according to claim 12,
- wherein at least one connection region (410, 420), the foot part (200) and/or the calf part (300) comprises two or more tongues (412, 422) and/or two or more grooves (212, 312), such that from a disconnected state at least one tongue (412, 422) can be interposed between two grooves (212, 312), or at least one groove (212, 312) can be interposed between two tongues (412, 422), and can be connected by relative rotation.

14. Ankle-foot-orthosis (100) according to one of claims 10 to 13,
- wherein the first connection region (410) and the foot part (200) are held in a static relative state by friction,
and/or
- wherein the second connection region (420) and the calf part (300) are held in a static relative state by friction.

15. Ankle-foot-orthosis (100) according to one of claims 10 to 14,
- wherein the first connection region (410) and the foot part (200) are held in a static relative state by one or more removable pins and/or pads (500),
and/or
- wherein the second connection region (420) and the calf part (300) are held in a static relative state by one or more removable pins and/or pads (500).
